# EUROPEAN PATENT APPLICATION

(11) **EP 1 977 742 A1**
(43) Date of publication of application: **08.10.2008**
(21) Application number: 07706289.1
(22) Date of filing: 30.01.2007
(51) Int. Cl.: A61K 31/191, A23L 1/30, A61K 31/365, A61P 13/04

(54) **PREVENTIVE/REMEDY FOR URINARY CALCULOSIS**

(30) Priority: 31.01.2006 JP 2006022482
(71) Applicant: Godo Shusei Co., Ltd., Tokyo, 1048162 (JP)
(72) Inventor: SANAI, Kazuko, Matsudo-shi, Chiba 271-0064 (JP); TANAKA, Yuko, Matsudo-shi, Chiba 271-0064 (JP)
(74) Representative: Hartz, Nikolai
(86) International application number: PCT/JP2007/000040
(87) International publication number: WO 2007/088704

(57) **Abstract**

The present invention relates to a prophylactic/therapeutic drug for urolithiasis containing as an active ingredient L-aldonic acid, a salt thereof, or a lactone form thereof.

## Description

### Technical Field

The present invention relates to a prophylactic or therapeutic drug for urolithiasis, and more particularly to a prophylactic or therapeutic drug for urolithiasis involving calcium oxalate calculi.

### Background Art

Urolithiasis is a disease characterized by severe pain in the abdomen as a main symptom caused by formation of calculi at any site of the urinary tract ranging from a kidney to the urethra. In Japan, over one hundred thousand of people are thought to suffer from urolithiasis, and the annual incidence rate thereof increases year by year. The incidence rate is higher in young generation, as the life-style of the young generation including in their diet has been changed. Since formation of urinary calculi tends to recur, prevention of the recurrence is a key issue not only from a medical aspect and but also from an economical aspect (Non-Patent Document 1).

Currently, the treatment of urolithiasis is performed by crashing calculi by shock wave, promoting spontaneous excretion of calculi, a drug therapy, etc. When calculi are crushed by shock wave, fragments of calculi remain in the urinary tract, which is problematic, and calculi often form again (Non-Patent Document 1). Thus, demand has arisen for the development of an excellent drug for urolithiasis.

Clinically, most (80%) of the cases of human urinary calculi are considered calcium oxalate calculi and calcium phosphate calculi (Non-Patent Document 2). Currently, there is no drug effective for the prevention and treatment of urolithiasis involving calcium oxalate calculi, and a diet therapy is generally employed. In such a diet therapy, patients are advised to follow, for example, (1) to prevent excessive consumption of food having high oxalic acid content or (2) to take sufficient amount of calcium so as to prevent aggravation of calculi which would otherwise be caused by promoted absorption of oxalic acid under insufficient calcium conditions. However, diet therapies cannot effectively prevent recurrence of formation of calculi (Non-Patent Documents 1 and 3).

Meanwhile, D-gluconic acid is known to dissolve calcium phosphate calculi and magnesium phosphate calculi, but not to dissolve calcium oxalate calculi (Non-Patent Documents 4 to 6).
Non-Patent Document 1: Urolithiasis treatment guidelines (Kanehara Co., Ltd., Tokyo, 2002)
Non-Patent Document 2: Journal of Japanese Urological Association 96; 41, 2005
Non-Patent Document 3: Hinyo-Kiyo 50; 591-596, 2004
Non-Patent Document 4: Urologia Internationalis (1998), 61(4), 210-214
Non-Patent Document 5: Klinische Wochenschrift (1962), 40, 594-597
Non-Patent Document 6: Northwest Medicine (1943), 42, 226-229

### Disclosure of the Invention

### Problems to be Solved by the Invention

An object of the present invention is to provide a prophylactic or therapeutic drug effective for urolithiasis, particularly urolithiasis involving calcium oxalate calculi. Means for Solving the Problems

In view of the foregoing, the present inventors have investigated a variety of drugs in terms of the effect for treating calcium oxalate urolithiasis by use of model rats to which ethylene glycol(EG) has been continuously administered (i.e., calcium oxalate urolithiasis model rats). As a result, quite surprisingly, the inventors have found that L-aldonic acid, a salt thereof, or a lactone form thereof, particularly L-arabonic acid, exhibits an excellent oxalic acid excretion-promoting effect and an effect of reducing the oxalic acid level and the calcium level in the kidneys, to thereby serve as an effective substance for urolithiasis. The present invention has been accomplished on the basis of this finding.

Accordingly, the present invention provides a prophylactic or therapeutic drug and food for urolithiasis containing, as an active ingredient, L-aldonic acid, a salt thereof, or a lactone form thereof.
The present invention also provides use of L-aldonic acid, a salt thereof, or a lactone form thereof for producing a prophylactic or therapeutic drug and food for urolithiasis. The present invention also provides a method for prevention or treatment of urolithiasis, characterized by comprising administering to a subject in need thereof L-aldonic acid, a salt thereof, or a lactone form thereof.

### Effects of the Invention

L-aldonic acid exhibits an excellent effect of preventing and treating urolithiasis and high safety, and allows long-term intake. Therefore, the acid is a particularly useful pharmaceutical or food for preventing recurrence of urolithiasis. In addition, L-aldonic acid hardly gives adverse side effect, since virtually 100% of the acid taken by a subject is excreted to urine.

### Brief Description of the Drawings

[Fig. 1] A graph showing body weight changes in the normal group, urolithiasis group, and arabonic acid salt-administered group.
[Fig. 2] A photograph showing left and right kidneys of the respective groups after the experiment.
[Fig. 3] A graph showing changed in blood L-arabonic acid level of rats after intravenous administration of sodium L-arabonate.

### Best Modes for Carrying Out the Invention

The L-aldonic acid employed in the present invention may be an oxidized product of L-aldose. Examples of the L-aldose include L-glyceraldehyde, L-threose, L-erythrose, L-lyxose, L-xylose, L-arabinose, L-ribose, L-talose, L-galactose, L-idose, L-gulose, L-mannose, L-glucose, L-altrose, L-allose, and L-glycero-D-manno-heptose. Oxidized products of L-fucose, L-rhamnose, etc. may also be employed. The thus-produced L-aldonic acids are present with the corresponding lactone forms in an equilibrium state. Among these L-aldonic acids, L-arabonic acid is particularly preferred.

Examples of the salt of L-aldonic acid include alkali metal salts such as sodium salts and potassium salts; alkaline earth metal salts such as calcium salts and magnesium salts; and metal salts such as copper salts, iron salts, and zinc salts. Of these, alkali metal salts such as sodium salts and potassium salts are particularly preferred.

In one exemplary method of producing L-arabonic acid, *Acinetobacter baumannii,* which is a microorganism capable of oxidizing aldose, is inoculated to a liquid medium containing L-arabinose, followed by culturing under stirring and air passage. As culturing proceeds, L-arabinose contained in the culture decreases, and L-arabonic acid forms. In this method, when calcium carbonate is added in advance to the culture medium, the pH of the culture can be stabilized, and a calcium salt of the formed L-arabonic acid is yielded. After L-arabinose contained in the medium has been completely consumed, the culture is filtered to thereby remove solid contents such as microorganism cells. The filtrate is purified through a known technique such as passage through an ion-exchange resin column, to thereby yield high-purity L-arabonic acid. Needless to say, an alkali metal salt of L-arabonic acid can be produced through a known technique.

In the present invention, use of L-arabonic acid, a salt thereof, or a lactone form thereof is particularly preferred, from the viewpoints of the effect of the urolithiasis treatment and safety.

As described in the Examples hereinbelow, L-aldonic acid, particularly L-arabonic acid, exhibits an excellent oxalic acid excretion-promoting effect and an effect of reducing the oxalic acid level and the calcium level in the kidneys, which are the effects observed in calcium oxalate urolithiasis model animals. Therefore, L-aldonic acid serves as a useful prophylactic/therapeutic drug and food for urolithiasis, preferably calcium urolithiasis, more preferably urolithiasis involving calcium oxalate calculi, particularly urolithiasis mainly caused by calcium oxalate calculi.
In addition, L-arabonic acid has high safety. Although absorbability of L-arabonic acid is low, virtually 100% of the absorbed L-arabonic acid is excreted to urine. Thus, it is considered that an adverse side effect other than the target action in the urinary tract is less exerted.

The target urolithiasis prevented or treated by the medicine or food according to the present invention is preferably urolithiasis involving calcium oxalate calculi.
In the urolithiasis according to the present invention, both of an upper urolithiasis which produces calculi in the kidney or urinary tract and a lower urolithiasis which produces calculi in the bladder and urethra.

The medicine of the present invention may be administered perorally or parenterally. However, oral administration is preferred. In other words, the medicine is preferably provided in the form of a peroral composition. Examples of the form of the composition include powder, granules, tablets, capsules, solution, and syrup. In drug preparation, a pharmacologically acceptable carrier such as an excipient, a disintegrant, a lubricant, a binder, a moistening agent, a dispersant, a preservative, a sweetening agent, a fragrant material, or a coating agent may be employed.
Examples of the parenteral administration means include intravenous administration, hypodermal administration, intradermal administration, intramuscular administration, administration via urinary tract, and percutaneous administration.
When administered through the urinary tract, the medicine may be topically administered through the urinary tract or in the form of bladder irrigation composition.

A possible form of the food according to the present invention is a functional food such as "food for specified health use." Thus, products of the functional food may be labeled with, for example, "for prevention of urolithiasis," or "those who care about urinary calculi." Examples of the form of such functional foods include beverage, powder, granules, capsules, sweets, and milk products.

The daily dose or amount of the medicine or food of the present invention is preferably 100 mg to 10 g (as L-aldonic acid), more preferably 0.5 g to 5 g, for an adult.

### Examples

The present invention will next be described in detail by way of examples.

### Example 1

### A. Method of experiment

In the experiment, 7-week-old male Wistar rats (Charles River Laboratories Japan, Inc.) were employed. The groups were treated as follows (see Table 1).

**[Table 1]**

| Experiment group | (n) | Treatment (fed water) | Experiment period |
|---|---|---|---|
| Normal group | (6) | Distilled water | 12 weeks |
| Urolithiasis group | (6) | 1% EG | 12 weeks |
| Arabonate salt-administered group | (6) | 1% EG+ 0.5% Arabonate salt | 12 weeks |

In Table 1, the term "arabonate salt" refers to an equilmole mixture of sodium L-arabonate and potassium L-arabonate (i.e., sodium L-arabonate (480 mg) and potassium L-arabonate (520 mg) in 1 g of mixture).

### Measurement items

During the experiment period, rats were placed in a metabolic cage, and body weight, chow intake, water intake, amount of urine, pH of urine, urinary calcium level, urinary sodium level, urinary potassium level, and urinary oxalic acid level were measured every two weeks. At the end of week 12, blood was collected from the heart of each rat under anesthesia with ether, and blood calcium level and blood oxalic acid level were determined. The kidneys (left and right) were removed from each rat, and weight of each kidney, calcium level in the kidney, and oxalic acid level in the kidney were measured.

### Measurement method

The urine samples were deproteinated with 1N perchloric acid (perchloric acid 1 vol. and urine 4 vol.) before measurement. Calcium concentration was determined by means of Calcium C Test Wako (product of Wako Pure Chemical Industries, Ltd.), and oxalic acid concentration was determined through HPLC.
In HPLC, a polymer column ICSep ICE-ION-300 for organic analysis (product of Transgenomic), a mobile phase of 0.0085N sulfuric acid, and an RI detector were employed. Blood samples were deproteinated with 1N perchloric acid (perchloric acid liquid 1 vol. and blood 1 vol.), and calcium concentration and oxalic acid concentration were determined in the same manner. The amount of calcium in a kidney was determined by ashing a portion of the kidney at 550°C for 18 hours, dissolving the ash in 1% HCl, and measuring Ca level by means of Calcium C Test Wako. The oxalic acid level in a kidney is determined by homogenizing a portion of a kidney with 1N perchloric acid and measuring the level through HPLC. The pH of urine samples was determined by means of a single-drop pH meter (product of Shindengen Electric manufacturing Co., Ltd.), and the urinary sodium level and urinary potassium level were determined by means of a compact ion-meter (product of Horiba, Ltd.).

### B. Results of experiment

### 1. Body weight, water intake, and urine amount

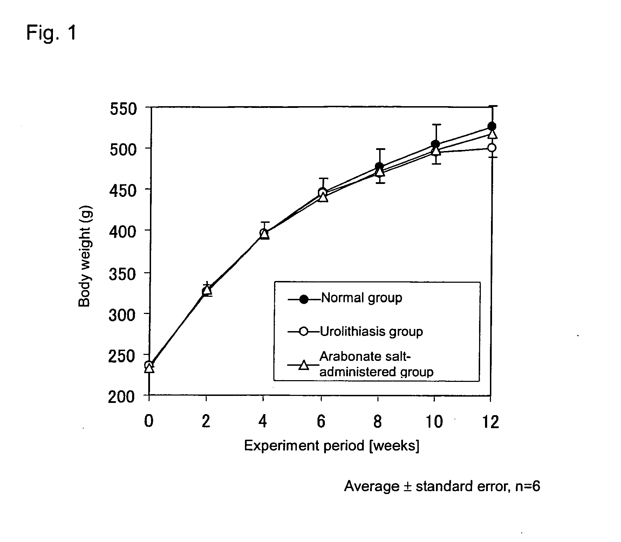

The disease group (EG-administered) (hereinafter referred to as urolithiasis group) and the arabonate salt-administered group exhibited a comparatively favorable increase in body weight. No difference was observed in the weight increasing tendency among the three groups including the normal group (Fig. 1). The water intake and urine amount were varied depending on the time point of measurement, but no difference was observed in the variation tendency among the three groups. Therefore, there is no difference in amount of administered EG between the urolithiasis group and the arabonate salt-administered group (Table 9).

2. Amounts of oxalic acid, calcium, sodium, and potassium which were excreted to urine, pH of urine, and levels of calcium and oxalic acid in kidneys, determined with respect to disease model rats
In the urolithiasis group, the amount of oxalic acid excreted to urine considerably increased from week 2 and was maintained at a high level 3 to 7 times that of the normal group to week 12 (p<0.01, Table 2). The pH of urine tended to decrease. At week 2 (p<0.01) and week 6 (p<0.05), the pH was significantly reduced as compared with that of the normal group (Table 3). The amount of calcium excreted to urine tended to increase, but the increase was not significant (Table 4). No significant increase was observed in the amount of excreted sodium until week 12 (Table 5). The amount of potassium excreted to urine tended to increase. At week 8 (p<0.05) and week 10 (p<0.01), the amount significantly increased as compared with the normal group (Table 6).
At the end of week 12, kidneys were removed from each rat through dissection. The kidney weight, oxalic acid level in the kidney, and calcium level in the kidney significantly increased 2-fold, 60-fold, and 800-fold, respectively, as compared with the normal group (p<0.01). The amount by mole of oxalic acid and that of calcium in a kidney were almost equivalent. The data indicate the progress of urolithiasis involving calcium oxalate (Table 7).

### 3. Amount of administered arabonate salt

The amount of an administered arabonate salt, calculated from the water intake, was 3.7 to 6.9 mmol/kg/day (Table 8).

### 4. Effect of arabonate salt

### 4-1. Amounts of oxalic acid, calcium, sodium, and potassium, excreted into urine and pH of urine

In the arabonate salt-administered group, the amount of oxalic acid excreted to urine tended to increase. At week 12 (p<0.05), the amount significantly increased as compared with the urolithiasis group (Table 2). The pH of urine was changed to a neutral value as observed in the arabonate salt-administered group. At week 2 (p<0.01), the pH significantly increased as compared with the urolithiasis group (Table 3). No effect of administration of arabonate salt was observed on the amount of calcium excreted to urine (Table 4). The amounts of sodium and potassium excreted to urine tended to increase. At week 8 (p<0.05) and week 10 (p<0.05), the sodium amount significantly increased as compared with the urolithiasis group (Table 5). At week 2 (p<0.01), week 4 (p<0.05), and week 10 (p<0.01), the potassium amount significantly increased as compared with the urolithiasis group (Table 6).

### 4-2. Appearance and weight of kidney, and amounts of calcium and oxalic acid in kidney

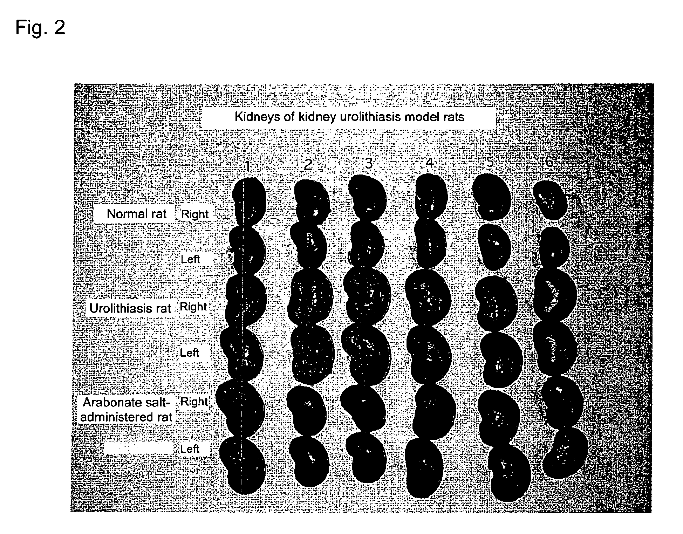

Fig. 2 is a photograph showing the appearance of kidneys of the rats of the all tested groups. In the urolithiasis group, six of the six tested rats exhibited hypertrophy and abnormal color of kidneys, with considerably varied degrees. In the arabonate salt-administered group, five of the six tested rats exhibited improvement of kidney appearance. One rat (No. 1) of the group could not be evaluated. In the arabonate salt-administered group, the weight of a kidney and the oxalic acid level in the kidney significantly decreased as compared with the urolithiasis group (p<0.05). Although the calcium level in the kidney was lower as compared with the urolithiasis group, the difference could not be evaluated with significance due to large variation in the data (Table 7).

**[Table 2]**

| Amount (µmol) of oxalic acid excreted into urine for 24 hours during experiment period | | | | | | | | |
|---|---|---|---|---|---|---|---|---|
| | n | 0w | 2w | 4w | 6w | 8w | 10w | 12w |
| Normal group | 6 | 59±6.7 | 40±18.3 | 72±5.0 | 57±12.5 | 69±9.3 | 64±6.2 | 52±5.2 |
| Urolithiasis group | 6 | 62±6.4 | 278±22.2** | 280±12.2** | 247±6.6** | 230±10.8** | 230±13.2** | 162±20.7** |
| Arabonate salt-administered group | 6 | 60±7.5 | 261±32.3 | 299±24.2 | 272±17.7 | 259±23.5 | 264±16.9 | 225±15.3⁺ |

| | | | | | | | | |
|---|---|---|---|---|---|---|---|---|
| av. ± standard error, **: P<0.01 vs. normal group, +: P<0.05 vs. urolithiasis group | | | | | | | | |

**[Table 3]**

| pH of urine (24 hours) during experiment period | | | | | | | | |
|---|---|---|---|---|---|---|---|---|
| | n. | 0w | 2w | 4w | 6w | 8w | 10w | 12w |
| Normal group | 6 | 6.9±0.07 | 7.3±0.15 | 7.4±0.30 | 7.9±0.22 | 8.5±0.25 | 8.2±0.37 | 8.5±0.29 |
| Urolithiasis group | 6 | 7.0±0.09 | 6.1±0.15** | 6.7±0.41 | 6.9±0.39* | 6.9±0.66 | 7.3±0.58 | 7.8±0.59 |
| Arabonate salt-administered group | 6 | 7.0±0.06 | 6.9±0.11⁺⁺ | 7.1±0.16 | 7.4±0.25 | 7.6±0.35 | 7.3±0.42 | 7.3±0.18 |

| | | | | | | | | |
|---|---|---|---|---|---|---|---|---|
| av. ± standard error, *: P<0.05, **: P<0.01 vs. normal group, ++: P<0.01 vs. urolithiasis group | | | | | | | | |

**[Table 4]**

| Amount (mg) of Ca excreted into urine for 24 hours during experiment period | | | | | | | | |
|---|---|---|---|---|---|---|---|---|
| | n | 0w | 2w | 4w | 6w | 8w | 10w | 12w |
| Normal group | 6 | 1.7±0.32 | 0.9±0.24 | 0.8±0.14 | 0.7±0.16 | 0.4±0.14 | 0.7±0.16 | 0.8±0.14 |
| Urolithiasis group | 6 | 1.3±0.27 | 1.1±0.12 | 0.9±0.14 | 0.8±0.11 | 0.7±0.12 | 0.8±0.12 | 0.7±0.15 |
| Arabonate salt-administered group | 6 | 1.1±0.23 | 0.9±0.18 | 1.0±0.15 | 0.9±0.24 | 0.7±0.13 | 0.9±0.25 | 0.9±0.18 |

| | | | | | | | | |
|---|---|---|---|---|---|---|---|---|
| av. ± standard error | | | | | | | | |

**[Table 5]**

| Amount (mg) of Na excreted into urine for 24 hours during experiment period | | | | | | | | |
|---|---|---|---|---|---|---|---|---|
| | n | 0w | 2w | 4w | 6w | 8w | 10w | 12w |
| Normal group | 6 | 20±0.9 | 23±1.3 | 20±1.7 | 20±4.3 | 12±1.4 | 12±2.2 | 22±11.7 |
| Urolithiasis group | 6 | 22±1.9 | 24±1.8 | 22±2.2 | 21±1.1 | 16±1.3 | 17±1.3 | 17±2.2 |
| Arabonate salt-administered group | 6 | 18±1.9 | 34±4.4 | 28±2.6 | 25±3.0 | 24±3.0⁺ | 25±3.0⁺ | 21±1.2 |

| | | | | | | | | |
|---|---|---|---|---|---|---|---|---|
| av. ± standard error, +: P<0.01 vs. urolithiasis group | | | | | | | | |

**[Table 6]**

| Amount (mg) of K excreted into urine for 24 hours during experiment period | | | | | | | | |
|---|---|---|---|---|---|---|---|---|
| | n | 0w | 2w | 4w | 6w | 8w | 10w | 12w |
| Normal group | 6 | 32±2.2 | 30±3.1 | 33±5.9 | 37±9.3 | 12±2.6 | 13±2.7 | 19±8.8 |
| Urolithiasis group | 6 | 36±3.6 | 37±3.0 | 38±5.0 | 40±3.1 | 20±1.8* | 23±1.5** | 24±4.7 |
| Arabonate salt-administered group | 6 | 31±3.2 | 57±3.1⁺⁺ | 51±2.7⁺ | 42±6.6 | 28±5.2 | 37±3.6⁺⁺ | 33±3.0 |

| | | | | | | | | |
|---|---|---|---|---|---|---|---|---|
| av. ± standard error, *: P<0.05, **: P<0.01 vs. normal group, +: p<0.05, ++: P<0.01 vs. urolithiasis group | | | | | | | | |

**[Table 7]**

| Kidney weight, Ca level in the kidney, and oxalic acid level in the kidney | | | | |
|---|---|---|---|---|
| | n | Weight (g) | Ca level (µmol) | Oxalic acid level (µmol) |
| Normal group | 6 | 3.1±0.19 | 8.2±0.46 | 110.0±6.60 |
| Urolithiasis group | 6 | 6.6±0.35** | 6641.3±1536.36** | 6615.9±982.67** |
| Arabonate salt-administered group | 6 | 5.2±0.48⁺ | 3386.9±1088.72 | 3461.2±977.94⁺ |

| | | | | |
|---|---|---|---|---|
| av. ± standard error, **: P<0.01 vs. normal group, +: P<0.05 vs. urolithiasis group | | | | |

**[Table 8]**

| Na L-arabonate intake and K L-arabonate intake of arabonate salt-administered group (mmol/kg/day) | | | | | | | | |
|---|---|---|---|---|---|---|---|---|
| | n | 0w | 2w | 4w | 6w | 8w | 10w | 12w |
| Na arabonate | 6 | 0 | 3.44±0.666 | 2.25±0.453 | 1.86±0.315 | 2.56±0.468 | 2.28±0.406 | 1.89±0.222 |
| K arabonate | 6 | 0 | 3.44±0.665 | 2.25±0.452 | 1.85±0.315 | 2.56±0.467 | 2.27±0.406 | 1.89±0.222 |

| | | | | | | | | |
|---|---|---|---|---|---|---|---|---|
| av. ± standard error | | | | | | | | |

**[Table 9]**

| Ethylene glycol (EG) intake during experiment period (mL/kg/day) | | | | | | | | |
|---|---|---|---|---|---|---|---|---|
| | n | 0w | 2w | 4w | 6w | 8w | 10w | 12w |
| Normal group | 6 | 0 | 0 | 0 | 0 | 0 | 0 | 0 |
| Urolithiasis group | 6 | 0 | 3.00±0.309 | 1.82±0.215 | 1.50±0.225 | 1.98±0.287 | 1.67±0.205 | 1.25±0.112 |
| Arabonate salt-administered group | 6 | 0 | 2.70±0.522 | 1.76±0.355 | 1.45±0.247 | 2.01±0.367 | 1.78±0.318 | 1.48±0.174 |

| | | | | | | | | |
|---|---|---|---|---|---|---|---|---|
| av. ± standard error | | | | | | | | |

The effect of administration of arabonate salt was studied through employment of calcium oxalate urolithiasis model rats. The results are as follows.
(1) After administration of an arabonate salt, significant increases were observed in the amount of oxalic acid excreted to urine (week 12 (p<0.05)), the amount of sodium excreted to urine (week 8 (p<0.05) and week 10 (p<0.05)), and the amount of potassium excreted to urine (week 2 (p<0.01), week 4 (p<0.05), and week 10 (p<0.01)).
(2) After administration of an arabonate salt, five of the six rats exhibited improved appearance of the kidney, and the weight of the kidney and the oxalic acid level in the kidney significantly decreased (p<0.05). In addition, the calcium level in the kidney was also improved. Currently, there is no such medicine having the above action; an arabonate salt was found to serve as an effective therapeutic drug for calcium oxalate urolithiasis.

### Example 2

Blood L-arabonic acid level and percent excretion of L-arabonic acid to urine (24 hours) of rats after peroral administration of the acid
In the experiment, 6-week-old male Wistar rats were employed after fasting overnight. To each rat, L-arabonic acid (5 mmol/kg) was orally administered, and the rats were placed in a metabolic cage where urine was collected for 24 hours. Blood collection was performed at 30 minutes, 1 hour, 2 hours, and 6 hours after administration through the tail vein. After removal of protein from the blood samples and urine samples, the blood L-arabonic acid level of each sample was determined through HPLC. Instead of L-arabonic acid, D-gluconic acid was administered to the rats of the control group. The blood D-gluconic acid level was determined by means of an F-kit (product of Roche Diagnostic).

**[Table 10]**

| Blood level of rats after oral administration of L-arabonic acid (µmol/mL) | | |
|---|---|---|
| | L-Arabonic acid 5 mmol/kg p.o. | D-gluconic acid 5 mmol/kg p.o. |
| 30 minutes | 0.26±0.257 | 0.16±0.027 |
| 1 hour | 0.76±0.082 | 0.24±0.046 |
| 2 hours | 0.15±0.088 | 0.35±0.070 |
| 6 hours | 0.04±0.037 | 0.07±0.005 |

| | | |
|---|---|---|
| av. ± standard error | | |

**[Table 11]**

| Percent urine excretion (24 hours) of rats after oral administration of L-arabonic acid | | |
|---|---|---|
| | L-Arabonic acid 5 mmol/kg p.o. | D-gluconic acid 5 mmol/kg p.o. |
| Percent urine excretion (24 hours) (%) | 8.8±2.54 | 0 |

| | | |
|---|---|---|
| av. ± standard error | | |

As shown in Tables 10 and 11, after peroral administration of L-arabonic acid (5 mmol/kg), the blood L-arabonic acid level reached a peak value one hour after administration. The 24-hour amount of the acid excreted to urine was found to be about 9%, indicating that L-arabonic acid was absorbed by rats to a certain extent. Meanwhile, the blood D-gluconic acid level after peroral administration thereof was found to be lower than that of L-arabonic acid, and no excretion of D-gluconic acid to urine was observed.

### Example 3

### Test of safety of L-arabonic acid

Through a single-dose peroral experiment of mice, the LD₅₀ of L-arabonic acid was found to be about 10 g/kg, indicating that L-arabonic acid is a high-safety substance.

### Example 4

Blood sodium L-arabonate level and percent excretion of sodium L-arabonate to urine (24 hours) of rats after intravenous administration of the salt
(1) Method of experiment
   After fasting overnight, 10- to 11-week old Wistar male rats were anesthetized with Nembutal, and sodium L-arabonate (0.5 mmol/kg) was administered through the femoral vein of each rat. Blood was sampled through the carotid artery as time elapsed, whereby the L-arabonic acid level was determined. Blood collection was performed by means of a heparin-treated capillary before administration and 5 min, 15 min, 30 min, and 1 hour after administration.
   The percent urine excretion (24 hours) was determined in the following manner. To each of the 5- to 6-week-old male Wistar rats which had been fasted overnight, sodium L-arabonate (0.5 mmol/kg) was administered through the tail vein. The rats were placed in a metabolic cage where urine was collected for 24 hours. After removal of protein from the blood samples and urine samples, the blood L-arabonic acid level of each sample was determined through HPLC. Instead of L-arabonic acid, sodium D-gluconate was administered to the rats of the control group. The blood sodium D-gluconate level was determined by means of an F-kit (product of Roche Diagnostic).

**[Table 12]**

| Percent urine excretion (24 hours) of rats after intravenous administration of sodium L-arabonate | | |
|---|---|---|
| | Sodium L-arabonate 0.5 mmol/kg i.v. n=6 | Sodium D-gluconate 0.5 mmol/kg i.v. n=5 |
| Percent urine excretion (24 hours) (%) | 105.8±11.48 | 9.8±9.76 |

| | | |
|---|---|---|
| av. ± standard error | | |

### (2) Results of experiment

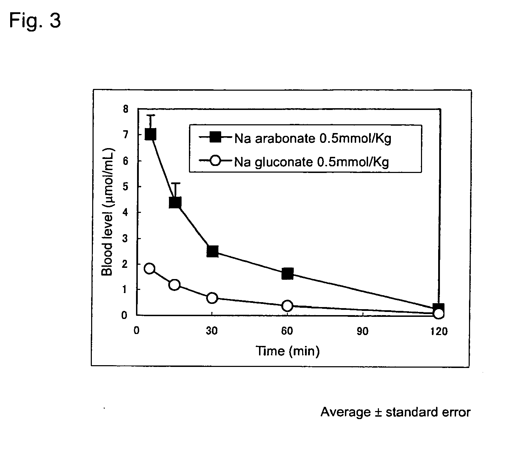

As shown in Fig. 3, the blood L-arabonic acid level five minutes after intravenous administration of sodium L-arabonate (0.5 mmol/kg) was 7.0 ± 0.72 µmol/mL, which decreased to about the half value 22 minutes after administration. L-Arabonic acid virtually disappeared from blood 2 hours after administration. In contrast, the blood D-gluconic acid level of the rats in the sodium D-gluconate-administered group was considerably low.
As shown in Table 12, almost 100% of sodium L-arabonate which had been intravenously administered was excreted to urine within 24 hours from administration. In contrast, only about 10% of sodium D-gluconate was excreted to urine for 24 hours, and a large portion thereof is thought to be metabolized and consumed in the body.
As described hereinabove, differing from D-gluconic acid, L-arabonic acid is not metabolized in the body and is immediately excreted to urine.

## Claims

1. A prophylactic/therapeutic drug for urolithiasis containing as an active ingredient L-aldonic acid, a salt thereof, or a lactone form thereof.

2. The prophylactic/therapeutic drug for urolithiasis as described in claim 1, wherein the L-aldonic acid is L-arabonic acid.

3. The prophylactic/therapeutic drug for urolithiasis as described in claim 1 or 2, wherein the urolithiasis is calcium urolithiasis.

4. The prophylactic/therapeutic drug for urolithiasis as described in claim 1 or 2, wherein the urolithiasis is urolithiasis involving calcium oxalate calculi.

5. The prophylactic/therapeutic food for urolithiasis containing as an active ingredient L-aldonic acid, a salt thereof, or a lactone form thereof.

6. The prophylactic/therapeutic food for urolithiasis as described in claim 5, wherein the L-aldonic acid is L-arabonic acid.

7. The prophylactic/therapeutic food for urolithiasis as described in claim 5 or 6, wherein the urolithiasis is calcium urolithiasis.

8. The prophylactic/therapeutic food for urolithiasis as described in claim 5 or 6, wherein the urolithiasis is urolithiasis involving calcium oxalate calculi.

9. Use of L-aldonic acid, a salt thereof, or a lactone form thereof for producing a prophylactic/therapeutic drug for urolithiasis.

10. Use as described in claim 9, wherein the L-aldonic acid is L-arabonic acid.

11. Use as described in claim 9 or 10, wherein the urolithiasis is calcium urolithiasis.

12. Use as described in claim 9 or 10, wherein the urolithiasis is urolithiasis involving calcium oxalate calculi.

13. Use of L-aldonic acid, a salt thereof, or a lactone form thereof for producing a prophylactic/therapeutic food for urolithiasis.

14. Use as described in claim 13, wherein the L-aldonic acid is L-arabonic acid.

15. Use as described in claim 13 or 14, wherein the urolithiasis is calcium urolithiasis.

16. Use as described in claim 13 or 14, wherein the urolithiasis is urolithiasis involving calcium oxalate calculi.

17. A method for prevention and treatment of urolithiasis, **characterized by** comprising administering to a subject in need thereof L-aldonic acid, a salt thereof, or a lactone form thereof.

18. The method as described in claim 17, wherein the L-aldonic acid is L-arabonic acid.

19. The method as described in claim 17 or 18, wherein the urolithiasis is calcium urolithiasis.

20. The method as described in claim 17 or 18, wherein the urolithiasis is urolithiasis involving calcium oxalate calculi.

21. The method as described in claim 17 or 18, wherein the mode of administration is oral administration.

22. The method as described in claim 17 or 18, wherein the mode of administration is administration of food.
